# EUROPEAN PATENT SPECIFICATION

(11) **EP 3 191 987 B1**
(45) Date of publication and mention of the grant of the patent: **25.12.2019**
(21) Application number: 15766392.3
(22) Date of filing: 11.09.2015
(51) Int. Cl.: G16H 40/63, G16H 50/50, A61B 5/08, A61M 16/00

(54) **A MECHANICAL VENTILATION SYSTEM FOR RESPIRATION WITH DECISION SUPPORT**
MECHANISCHES BELÜFTUNGSSYSTEM ZUR BEATMUNG MIT ENTSCHEIDUNGSUNTERSTÜTZUNG
SYSTÈME DE VENTILATION MÉCANIQUE POUR LA RESPIRATION DOTÉ D'UNE AIDE À LA DÉCISION

(30) Priority: 12.09.2014 DK 201470565
(43) Date of publication of application: 19.07.2017
(73) Proprietor: Mermaid Care A/S, 9400 Nørresundby (DK)
(72) Inventor: KARBING, Dan Stieper, 9000 Aalborg (DK); REES, Stephen Edward, 9260 Gistrup (DK); JENSEN, Jakob Bredal, 9000 Aalborg (DK)
(74) Representative: Høiberg P/S
(86) International application number: PCT/DK2015/050271
(87) International publication number: WO 2016/037627

(56) References cited:
- US-A1- 2001 004 893
- US-A1- 2005 098 178
- REES S E ET AL: "Using physiological models and decision theory for selecting appropriate ventilator settings", JOURNAL OF CLINICAL MONITORING AND COMPUTING, KLUWER ACADEMIC PUBLISHERS, DO, vol. 20, no. 6, 15 September 2006 (2006-09-15), pages 421-429, XP019451338, ISSN: 1573-2614, DOI: 10.1007/S10877-006-9049-5 cited in the application
- STEPHEN E REES ET AL: "The Intelligent Ventilator Project: Application of Physiological Models in Decision Support", 2 July 2011 (2011-07-02), ARTIFICIAL INTELLIGENCE IN MEDICINE, SPRINGER BERLIN HEIDELBERG, BERLIN, HEIDELBERG, PAGE(S) 149 - 158, XP019164371, ISBN: 978-3-642-22217-7 abstract page 150, paragraph 2 - page 154 page 155, paragraph 3
- FLEUR T TEHRANI ET AL: "A model-based decision support system for critiquing mechanical ventilation treatments", JOURNAL OF CLINICAL MONITORING AND COMPUTING, KLUWER ACADEMIC PUBLISHERS, DO, vol. 26, no. 3, 25 April 2012 (2012-04-25) , pages 207-215, XP035053371, ISSN: 1573-2614, DOI: 10.1007/S10877-012-9362-0
- DAS A ET AL: "Optimization of Mechanical Ventilator Settings for Pulmonary Disease States", IEEE TRANSACTIONS ON BIOMEDICAL ENGINEERING, IEEE SERVICE CENTER, PISCATAWAY, NJ, USA, vol. 60, no. 6, 1 June 2013 (2013-06-01), pages 1599-1607, XP011522663, ISSN: 0018-9294, DOI: 10.1109/TBME.2013.2239645

## Description

### Technical field of the invention

The present invention relates to a mechanical ventilation system for respiration of an associated patient with decision support for lung ventilator settings. In particular, the present invention relates to a decision support system aiding decisions related to lung ventilator settings, which may adapt to the patient's changing physiology upon changing ventilator settings. The invention also relates to a corresponding method for operating a mechanical ventilation system, and corresponding computer programme product for operating a mechanical ventilation system when executed on a suitable computer.

### Background of the invention

Patients residing at the intensive care unit typically receive mechanical support for their ventilation. Selecting the appropriate level of mechanical ventilation is not trivial, and it has been shown that appropriate settings can reduce mortality [1] (cf. reference list at the end of the description).

A challenge with the settings of a mechanical ventilator is that each setting may be beneficial for one physiological parameter of the patient but negative for another physiological parameter. Currently the clinician may get help by ventilator screens, extra devices monitoring physiological parameters (capnograph, pulse oximeter, monitor, etc.) and alarm settings if something is wrong.

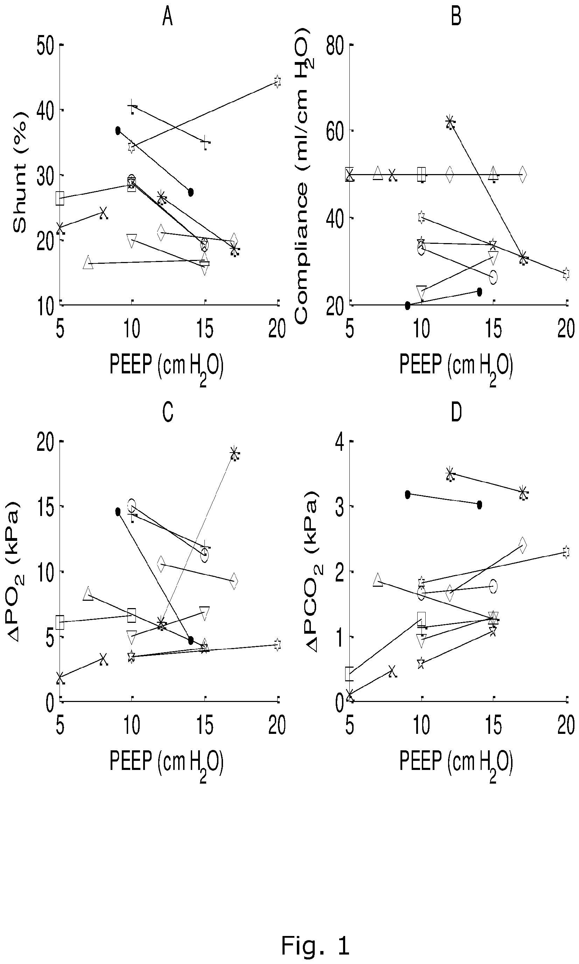

Decision support systems have been developed for minimizing failure in ventilator settings [2-5], with one system also developed for using models of patient physiology and clinical preferences. However, previous decision support systems are based on the assumption that patient physiological parameters remains constant when ventilator settings are changed. This is often the case for small changes in ventilator settings such as inspired oxygen and respiratory rate but not the case when large changes in ventilator settings are performed, or when settings such as Positive End Expiratory Pressure (PEEP) is modified. For example, PEEP affects blood flow, as well as the mechanical and gas exchange properties of the alveoli, the small units of the lungs where gas exchange takes place [6]. Patients may unfortunately respond very differently to changes in PEEP, and it is not possible to predict from measurement at one set of ventilator settings how a patient will respond to changes in PEEP. Patient responses to PEEP changes range from improvement in gas exchange and/or blood flow and/or lung mechanics to adverse effects on one or all of these aspects of lung physiology (see for example changes in some gas exchange and lung mechanics parameters, figure 1 clearly demonstrates this problem). If a decision support system is not able to adapt to such responses and provides advice based on a wrong prediction, the provided advice is likely to lead to worsening in patient outcome.

WO97/20592 (to Cardiopulmonary Corp., USA) discloses a ventilator control system controlling a ventilator pneumatic system in a medical ventilator. A simulator may be provided for predicting the status of the patient's pulmonary system prior to adjusting the control. The simulator applied could predict breathing patterns, but apparently not based on any deeper insight into patient physiology. Document "Using physiological models and decision theory for selecting appropriate ventilator settings" discloses a device according to the preamble of claim 1.

Hence, an improved mechanical ventilation system with decision support for lung ventilator settings and/or a corresponding system for decision support for mechanical ventilation, which are able to adapt to changes in patient physiology in response to changes in mechanical ventilation would be advantageous.

### Object of the invention

It is a further object of the present invention to provide an alternative to the prior art.

In particular, it may be seen as an object of the present invention to provide a mechanical ventilation system with decision support that solves the above mentioned problems of the prior art with adjusting ventilator settings, and particularly the uncertainty regarding how a patient will respond to changes in PEEP.

### Summary of the invention

Thus, the above described object and several other objects are intended to be obtained in a first aspect of the invention by providing a mechanical ventilation system with decision support for lung ventilator settings, which can particularly adapt to changes in patient physiology in response to changes in mechanical ventilator settings and adjust the decision support accordingly.

In a first aspect, the present invention relates to a mechanical ventilation system for respiration of an associated patient, the system being adapted for providing decision support for mechanical ventilation, the system comprising:
- ventilation means capable of mechanical ventilating said patient with air and/or one or more medical gases, the ventilation means having a plurality of settings (V_SET) comprising a positive end expiratory pressure (PEEP) setting,
- control means, the ventilator means being controllable by said control means by operational connection thereto, and
- measurement means arranged for measuring the inspired gas and/or the respiratory feedback of said patient in the expired gas in response to the mechanical ventilation, the measurement means being capable of delivering first data (D1) to said control means,
wherein the control means is adapted for using both the first data (D1) and second data (D2) indicative of the respiratory feedback in the blood in physiological models (MOD) descriptive of, at least, lung mechanics, and/or gas exchange in the lungs of the patient, the physiological models comprising a number of model parameters (MOD_P), and
wherein the control means is further arranged for simulating the effect on one, or more, model parameters (MOD_P) selected from lung mechanics parameters, gas exchange parameters, blood-acid-base parameters, respiratory drive parameters and circulation parameters of the physiological models for a suggested value of the positive end expiratory pressure (PEEP) setting for the ventilation means, and thereby provide decision support in relation to said suggested PEEP value.

The invention is particularly, but not exclusively, advantageous for providing mathematical based models of changes in physiology in response to changes in ventilator settings allowing prediction of changes in patient physiological parameters, thereby allowing mathematical physiological models to predict changes in clinical variables for a given value of PEEP. Thereby an additional abstraction level of the decision support system is included. This may then allow optimization of all ventilator settings across different levels of PEEP ultimately allowing the system an initial advice on optimal PEEP.

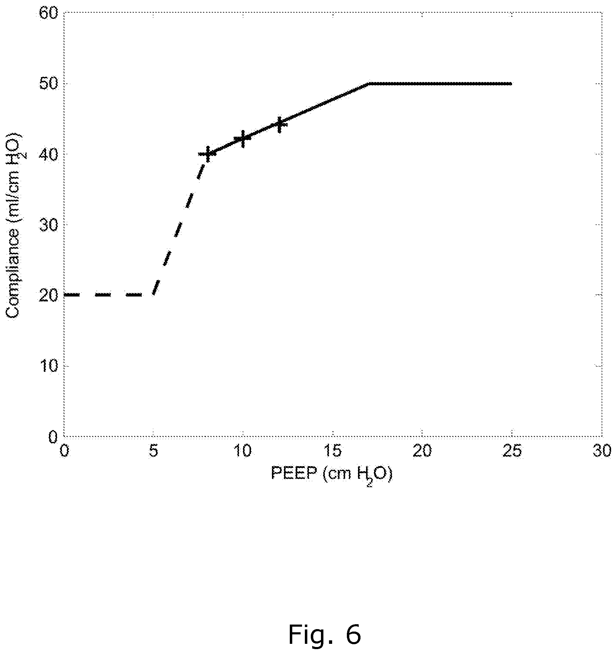

Because it is not possible to predict the optimal level of PEEP from one measurement point in time, PEEP is normally selected in a stepwise manner where PEEP is changed in small steps and the patient's response is monitored. Two overall strategies are applied in the clinical setting; lung recruitment (high airway pressure for a short time period) followed by stepwise PEEP reduction until the optimal balance is found, or stepwise increase in PEEP until optimal balance is found. As the two strategies depend on the inspiratory and expiratory lung mechanics, respectively, the resulting optimal PEEP may be different. The present invention, as a further novelty, therefore includes an algorithm for selection of appropriate physiological models for calculating optimal PEEP, such that the decision support system can provide advice for either of the two overall stepwise PEEP strategies (see figure 6 and the corresponding explanation below).

The advised level of PEEP and other ventilator settings may result in a different change in patient physiological parameters than expected, and initially described by the models. For example, a PEEP increase may recruit some lung regions (shunt improves, compliance improves), some of these regions may develop lower lobe atelectasis (low V/Q deterioration, worse oxygenation) and the increase in pressure may cause some lung regions to hyperinflate constricting blood vessels and consequently creating alveolar dead space where gas exchange is impaired (high V/Q deterioration, CO2 increase, pH decrease, compliance decrease). Alternatively, a PEEP increase can result in no recruitment with constant shunt, a decline in compliance and with significant increase in alveolar dead space representing a deleterious selection of PEEP. To overcome this problem, the present invention includes algorithms for learning the response of the patient and adapting the models of changes in physiological parameters to this response and update the decision support accordingly.

Some mathematical physiological models require experimental procedures for tuning the physiological parameters to the current patient status. This would be problematic during a stepwise strategy for finding optimal ventilator settings, as the repetitive performance of experimental procedures at each step would prolong finding the optimal ventilator settings. Therefore, as a further example the present invention may include a Bayesian or similar learning methods where model parameters used for calculating decision support are stepwise learned where values at previous steps are taken into account when learning the current value. Bayesian methods have previously been used for an initial estimation of model parameters [7] but not as a response to changing physiology.

It should be mentioned that the present invention may be implemented in connection with patients that are in fully supported ventilation mode, or assisted modes of ventilation. Alternatively, the present invention may be implemented in connection with patients that are in so-called mixed modes of ventilation i.e. combinations of fully controlled and supported modes of ventilation.

### Some term explanations are given below:

### Decision support:

In the present context the term "decision support" should be understood in the broadest sense of the term, decision support covers any means for assisting the user, here the clinician, in making the decision. This includes organization, integration and presentation of data as well as providing suggestions for rational decisions. Conventionally, decision support systems (DSS) may be either passive, active or cooperative in the interaction with the user of the DSS.

### Physiological model:

In the present context the term "physiological model" should be understood as the broadest sense of applied physiological models, the term physiological model here covering any means for linking clinically measurable values mathematically and for the individual patient, that is, that any parameters that needs tuning for the model to describe the individual patient's physiology should be possible to estimate from clinical measurements. In its simplest form such a physiological model can be a linear model of the equation y = a^{∗}x with a being the parameter of the model and y and x being clinical measureable values such as the link between pressure (x) and volume (y) with lung compliance (a) linking the two (Volume = Pressure^{∗}Compliance). The more complex end of the range include a model encompassing all chemical reaction equations of the buffer systems of human blood with parameters such as rate constants which may be assumed equal for all individuals but with for example Base Excess that should be tuned to the individual patient [8]. The invention requires at least physiological models of lung mechanics and/or gas exchange but may benefit greatly from models of blood acid-base status, respiratory drive and/or blood circulation etc.

### Lung Mechanics

A physiological model of lung mechanics links flows, volumes and or pressures which may be measured at the mouth, in the oesophagus, at the ventilator, in the ventilator tube, in the airways etc. Most importantly for the setting of PEEP is the pressure-volume relationship of the lungs which primarily depends on the compliance or elastance (=1/compliance) of the respiratory system. More complex physiological models may describe the resistances of the respiratory system and the individual elastic characteristics of the alveoli, the lung tissue, the diaphragm, the surfactant, the chest wall, the airways, etc. In control modes, identification of the overall characteristics of the respiratory system such as compliance is straightforward and is routinely measured by the ventilator. In support mode, the volumes inspired and expired by the patient not only depend on lung mechanics and ventilator settings, but also patient respiratory work. Simple models of lung mechanics in support ventilation may therefore describe an effective compliance, which is the combined pressure volume relationship of ventilator and patient work.

### Gas exchange

A physiological model of gas exchange links measurements of contents of inspired and expired gases to measured contents of these gases in blood (arterial, venous, mixed venous or capillary blood). Often this is mathematically implemented as a set of equations describing the exchange of one, or more, respiratory gases (oxygen, carbon dioxide, nitrogen) or inert gases added to the inspired gas (such as xenon, krypton, nitric oxide, methane etc.) across the alveolar membrane to capillary blood in one or more compartments and the mixing of the blood leaving these compartments with shunted venous blood to constitute the arterial blood, the latter which is often sampled and analysed in the clinical settings. Venous or mixed venous blood may also be sampled in the clinical setting and can also be described by a model by including equations with patient metabolism and blood flow in the model. Estimation of model parameters for gas exchange models, in particular for models with one or more of the aforementioned compartments, requires either invasive measurements such as a mixed venous blood sample from the pulmonary artery or that the gas contents of inspired gas is modified in one or more steps with the patient response to these changes measured continuously and/or at steady state this making it possible to separate the contributions of one or more mechanisms for gas exchange problems these in the model represented by parameters such as intrapulmonary shunt, ventilation/perfusion ratio etc. Gas exchange models often encompass a model of blood acid base status and the binding and transport of respiratory gases in blood.

### Blood acid-base and oxygen and carbon dioxide transport

A model of blood acid-base status and/or oxygen and carbon dioxide transport describes either empirically or by reaction equations some or all of the chemical reactions occurring in blood for chemical buffering and binding of gases to haemoglobin. The models may be in so-called steady state describing the reaction equations at equilibrium or dynamical describing the reactions over time. As such, the models link values of blood acid base that can be measured routinely at the bedside (such as oxygen and carbon dioxide pressures and pH) to values not easily obtained (such as base excess, concentrations of buffers, concentrations of respiratory gases etc.).

### Respiratory drive

A physiological model of respiratory drive links levels of oxygen, carbon dioxide and or hydrogen in blood and/or cerebrospinal fluid to the respiratory drive of the patient is the patient's ventilation. The patient's ventilation depends on the respiratory control center of the patient but also the condition of the respiratory muscles as well as any anaesthetics and analgesics given to the patient. The measured ventilation may therefore be different from the drive signal form the respiratory control center. A respiratory drive model may therefore also include in empirical or other form a representation of muscle strength and/or fatigue and any effects of anaesthetics and/or analgesics.

### Circulation

A physiological model of circulation relevant for PEEP links the effect of changes in mechanical ventilator settings and/or patient posture and/or patient fluid intake and status to the circulation of the patient as can be measured as cardiac output, pulse rate, stroke volume, cardiac resistance etc. or as can be assessed through changes in non circulatory values such as changes in gas exchange and lung volumes.

### Physiological parameters:

A physiological parameter should in this context be understood as a value describing the characteristics of a physiological system and can be assumed constant for changes in some clinical variables and/or ventilator settings. For example, pulmonary shunt describes the fraction of blood flow to the lungs not reaching ventilated alveoli representing the most important gas exchange problem in mechanically ventilated patients. Pulmonary shunt is known to remain constant for variation in a number of clinical variables and ventilator settings such as respiratory frequency and tidal volume.

Below are listed some physiological parameters suitable for application in the present invention: *Lung mechanics parameters* include lung compliance, respiratory resistances, respiratory system elastance, effective shunt, compliances and resistances of individual components of the respiratory system (alveoli, airways, chest wall, diaphragm, surfactant).

*Gas exchange parameters* include pulmonary shunt, venous admixture, effective shunt, Ventilation/perfusion (V/Q) ratio, degree of low V/Q, degree of high V/Q, ΔPO2, ΔPCO2, Arterial-end tidal O2 difference, Arterial-end tidal CO2 difference, anatomical dead space, alveolar dead space, physiological dead space, end-expiratory lung volume, functional residual capacity etc.

*Blood acid-base parameters* include base excess (BE), strong iron difference (SID), haemoglobin concentration, blood volume, rate constants for chemical reactions etc.

*Respiratory drive parameters* include central chemoreceptor threshold for oxygen and carbon dioxide, peripheral chemoreceptor threshold for oxygen and carbon dioxide, central chemoreceptor sensitivity for oxygen and carbon dioxide, peripheral chemoreceptor sensitivity for oxygen and carbon dioxide, strong ion difference for cerebrospinal fluid, muscle response factor, muscle fatigue, muscle strength etc.

*Circulation parameters* include cardiac output, vascular resistances, stroke volume, blood volume, venous return etc.

### Clinical outcome variables

A clinical outcome variable should in this context be understood in the broadest sense of the term describing any clinical measurable value, which can be associated with a clinical outcome and preference.

Clinical outcome variables can be categorised according to the relating aspect of respiratory physiology and can include all, but not exclusively or exhaustively, of the following lists:
*Lung mechanics outcome variables:* Including Peak pressure, Plateau pressure, peak inspiratory pressure, end expiratory pressure, peak flow, tidal volume, tidal volume per ml of predicted body weight, stress, strain, transpulmonary pressure, oesophageal pressure, abdominal pressure etc.

*Gas exchange outcome variables:* Including arterial, mixed venous, venous and capillary values of oxygen saturation, oxygen partial pressure, oxygen concentration, carbon dioxide partial pressure and carbon dioxide concentration.

*Blood acid-base outcome variables:* Including arterial, mixed venous, venous, capillary values of pH, hydrogen ion concentration, carbon dioxide partial pressure and concentration, bicarbonate concentration.

*Patient respiratory work outcome variables:* Including oxygen consumption, carbon dioxide production, changes in ventilation, respiratory rate, respiratory rate to tidal volume ratio, respiratory rate to tidal volume per kg predicted body weight ratio, work of breathing according to different formulae as that of Otis [9] or as assessed from changes in pressure-volume curves, electrical activity of respiratory muscles, electrical activity of respiratory muscles related to tidal volume or ventilation.

*Circulation outcome variables:* Including blood pressure, pulse rate, cardiac output, stroke volume, circulatory resistances, changes in lung volumes and/or gas exchange parameters and outcome variables with PEEP.

Below are given some examples of the present invention:
Advantageously, the control means may be arranged for simulating the effect on one, or more, model parameters (MOD_P) of the physiological models for a plurality of values (PEEP;1,...,n) of the positive end expiratory pressure setting for the ventilation means, and thereby provide decision support in relation to said plurality of PEEP values. This is particular advantageous because a clinician may thereby be given an improved overview of the possible PEEP values suitable. Preferably, the control means may be further arranged for suggesting an optimum value between the plurality of values of the positive end expiratory pressure setting for the ventilation means (PEEP;1,...,n) in order to guide the clinician.

In some examples, the control means may be further arranged for simulating the effect on one, or more, parameters (MOD_P) of the physiological models for one, or more, values in the positive end expiratory pressure setting for the ventilation means (PEEP) performed by a simulation based on at least two previous values of the PEEP setting for the ventilation means, optionally at least three, four or five values of PEEP setting, in order to improve the reliability and/or precision and/or the accuracy of simulation. In other variants, the control means may be further arranged for simulating the effect on one, or more, parameters (MOD_P) of the physiological models for one, or more, values in the positive end expiratory pressure setting for the ventilation means (PEEP) performed by a simulation based on at least two simulated values of the PEEP setting for the ventilation means, optionally at least three, four or five values of simulated PEEP setting, to improve the decision support available.

In some examples, the control means may comprise one, or more, positive end expiratory pressure (PEEP) models, each PEEP model comprising a model parameter (MOD_P) of a physiological model as a function of the PEEP settings for the ventilation means. These PEEP models then in turn allow use of physiological models to simulate the effect of PEEP on the effect of changes in other mechanical ventilator settings by input to models of for example gas exchange, lung mechanics, blood acid-base, circulation and metabolism. Beneficially, one, or more, of the PEEP models may be adapted to the patient, and/or the clinical condition of the patient, before initiating changes of the PEEP settings, and/or while changing the PEEP settings. Alternatively or additionally, the one, or more, PEEP models may be adapted to the patient by a learning module, the learning module having a set of a priori settings of PEEP model parameters (MOD_P_PEEP) based on the currently measured model parameter (MOD_P) value of the corresponding physiological models (MOD), preferably based on a Bayesian distribution based on patient type and/or clinical condition as it will be explained in more detail below.

In other examples, one, or more, of the physiological models (MOD) may be adapted to the patient, and/or the clinical condition of the patient, before initiating changes of the PEEP settings, and/or while changing the PEEP settings.

This may for instance improve the PEEP setting faster and/or in more reliable manner.

Advantageously, one, or more, additional physiological models (MOD) may be further descriptive of the metabolism of the patient, the blood circulation of the patient, acid-base status of the patient, oxygen and carbon dioxide transport for the patient, and/or the respiratory drive of the patient in order to provide a more complete model of the patient being mechanically ventilated. Preferably, at least two, three, four, or five, physiological models are integrated by having one, or more, variables in common. Thus, (outcome) variables such as saturation of arterial oxygen (SaO2) or pH in arterial blood (pHa) may be used in several physiological models of the present invention enabling a close integration of the physiological models.

In some examples, the control means may comprise one, or more, modules for choosing a PEEP changing strategy for the patient. In one variant, one choice of PEEP changing strategy may be based on an assumption of lung recruitment wherein a relatively high airway pressure is applied for a relatively short time followed by stepwise PEEP reduction until the optimal balance is reached. In another variant, one choice of PEEP changing strategy may be based on a stepwise increase of PEEP until an optimal balance is reached.

In a particular example, the control means may further comprise a plurality of clinical preference functions (CPFs) relating settings of positive end expiratory pressure (PEEP) for the ventilation means to a corresponding set of clinical outcome variables to connect the decision support with clinical practise and experience. Additionally or alternatively, clinical preference functions (CPFs) may - in the context of the present invention - be understood as a means for relating settings on the mechanical ventilator to a corresponding set of clinical outcome variables by associating said different values of said outcome variables with a level of preference. This may be implemented by a mathematical description of the clinically preferred values of said clinical outcome variables. For example, a level of oxygen saturation in the arterial blood of 95% is preferable to 94%, and 95% is therefore in the CPF associated with greater preference and this may be related to changing the inspired oxygen on the mechanical ventilator. As such, a CPF may allow the outcome to connect decision support with clinical practice and experience. Clinical preference functions (CPFs) may be formulated in many ways including in a non-exhaustive list: mathematical function (linear, exponential or other form), combination of several mathematical formulations, logical ruleset, mathematical weighing of outcome variables in calculation of decision support, and any combination of the aforementioned forms.

For example, the plurality of clinical preference functions (CPFs) may be chosen from the group consisting of: CPFs inserted by a clinician, CPFs chosen from a database of possible CPFs, CFPs a priori adapted to a specific patient based on general clinical input from a clinician, and CFPs a priori adapted to a specific patient according to patient needs, and any combinations and/or equivalents thereof.

A particular advantage of the present invention is that the plurality of clinical preference functions (CPFs) may be applied for providing decision support related to an overall optimisation of the PEEP setting of the mechanical ventilation for the patient.

In an another particular example, the positive end expiratory pressure (PEEP) setting may be further optimized with respect to other mechanical ventilation settings, preferably inspired oxygen (FIO2), tidal volume (VT), and/or pressure above PEEP, which is quite difficult to perform with previous methods for PEEP adjustment in clinical conditions.

In a second aspect, the present invention relates to a decision support system for providing decision support to an associated mechanical ventilation system for respiration aid of a patient, the mechanical ventilation system comprising:
- ventilation means capable of mechanical ventilating said patient with air and/or one or more medical gases, the ventilation means having a plurality of settings (V_SET) comprising a positive end expiratory pressure (PEEP) setting, and
- measurement means arranged for measuring the inspired gas and/or the respiratory feedback of said patient in the expired gas in response to the mechanical ventilation, the measurement means being capable of delivering first data (D1) to said decision support system,
wherein the decision support system is adapted for using both the first data (D1) and second data (D2) indicative of the respiratory feedback in the blood in physiological models (MOD) descriptive of, at least, lung mechanics, and/or gas exchange in the lungs of the patient, the physiological models comprising a number of model parameters (MOD_P), and
wherein the decision support system is further arranged for simulating the effect on one, or more, model parameters (MOD_P) selected from lung mechanics parameters, gas exchange parameters, blood-acid-base parameters, respiratory drive parameters and circulation parameters of the physiological models for a suggested value of the positive end expiratory pressure (PEEP) setting for the ventilation means, and thereby provide decision support in relation to said suggested PEEP value.

This aspect of the invention is particularly, but not exclusively, advantageous in that the present invention may be implemented by operating the decision support system together with an existing mechanical ventilation system, the mechanical ventilation system possibly requiring little or no modification to cooperate with the decision support system according to the present invention.

In a third aspect, the present invention relates to method for operating an mechanical ventilation system for respiration of an associated patient, the system being adapted for providing decision support for mechanical ventilation, the method comprising:
- providing ventilation means capable of mechanical ventilating said patient with air and/or one or more medical gases, the ventilation means having a plurality of settings (V_SET) comprising a positive end expiratory pressure (PEEP) setting,
- providing control means, the ventilator means being controllable by said control means by operational connection thereto, and
- providing measurement means arranged for measuring the inspired gas and/or the respiratory feedback of said patient in the expired gas in response to the mechanical ventilation, the measurement means being capable of delivering first data (D1) to said control means,
wherein the control means is adapted for using both the first data (D1) and second data (D2) indicative of the respiratory feedback in the blood in physiological models (MOD) descriptive of, at least, lung mechanics, and/or gas exchange in the lungs of the patient, the physiological models comprising a number of model parameters (MOD_P), and
wherein the control means is further arranged for simulating the effect on one, or more, model parameters (MOD_P) selected from lung mechanics parameters, gas exchange parameters, blood-acid-base parameters, respiratory drive parameters and circulation parameters of the physiological models for a suggested value of the positive end expiratory pressure (PEEP) setting for the ventilation means, and thereby provide decision support in relation to said suggested PEEP value.

In a fourth aspect, the invention relates to a computer program product being adapted to enable a computer system comprising at least one computer having data storage means in connection therewith to control an mechanical ventilation system as described in the third aspect.

This aspect of the invention is particularly, but not exclusively, advantageous in that the present invention may be accomplished by a computer program product enabling a computer system to carry out the operations of the mechanical ventilation system or the decision support system of the first and second aspect of the invention, respectively, when down- or uploaded into the computer system. Such a computer program product may be provided on any kind of computer readable medium, or through a network.

The individual aspects of the present invention may each be combined with any of the other aspects. These and other aspects of the invention will be apparent from the following description with reference to the described examples.

### Brief description of the figures

The invention will now be described in more detail with regard to the accompanying figures. The figures show one way of implementing the present invention and is not to be construed as being limiting to other possible embodiments falling within the scope of the attached claim set.
Figure 1 shows patient examples for various settings of PEEP and the corresponding changes in physiological parameters,
Figure 2 is a schematic drawing of a mechanical ventilation system according to the present invention,
Figure 3 is a drawing of the decision support for providing decision support to an associated mechanical ventilation system (not shown),
Figure 4 shows graphs of the physiological model parameters, ventilator settings, simulated patient changes, and clinical preference functions (CPF) as a function of the PEEP,
Figure 5 shows graphs of PEEP models with learning of gas exchange,
Figure 6 shows a graph of a PEEP model of lung compliance,
Figure 7 shows a graph of other PEEP models of lung mechanics and metabolism,
Figure 8 shows a flow chart with two PEEP changing strategies; recruitment or so-called crawl,
Figure 9 shows the timing of the two PEEP changing strategies, recruitment or so-called crawl, from Figure 8,
Figure 10 shows the learning of gas exchange model parameters applying a Bayesian approach,
Figure 11 shows a simulated patient case for a patient in a control ventilator mode where an example of the invention is applied to select PEEP over three steps using the crawl PEEP selection strategy, and
Figure 12 is a schematic system-chart representing an outline of operations of the method according to the invention.

### Detailed description of an example

Figure 1 shows patient examples for various settings of PEEP and the corresponding changes in physiological parameters. When changes in ventilator settings of PEEP is modified, patient physiological parameters change cause corresponding changes in some gas exchange and lung mechanics parameters as seen in figure 1, and mathematical models can no longer accurately predict changes in the these clinical variables. A decision support system using mathematical models for simulation and in calculation of optimal ventilator settings will then not be able to provide correct advice.

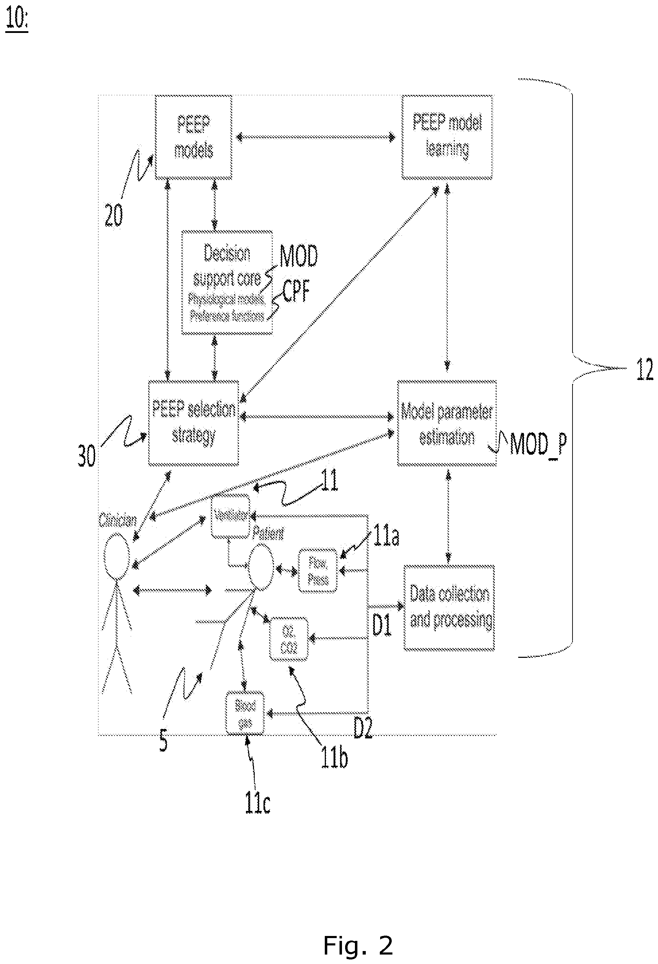

In one example for the mechanical ventilation system with decision support for PEEP setting, the invention can be implemented as illustrated in figure 2. The mechanical ventilation system or respiration of an associated patient 5 is further adapted for providing decision support for mechanical ventilation, the system comprising ventilation means 11 capable of mechanical ventilating said patient with air and/or one or more medical gases, .e.g. oxygen, the ventilation means having a plurality of settings, V_SET, comprising a positive end expiratory pressure (PEEP) setting.

Additionally, control means 12 are provided, the ventilator means being controllable by said control means by operational connection thereto. The control means may be integrated on a computer system operationally connected to the ventilation means 11 and the measurements 11a, 11b, and/or 11c.

Measurement means, 11a, and 11b, are arranged for measuring the inspired gas and/or the respiratory feedback of said patient in the expired gas in response to the mechanical ventilation, the measurement means being capable of delivering first data D1 to said control means. The measurement means are shown as separate entities but could alternatively form part of the ventilator means 11 without significantly change the basic principle of the present invention. The measurement means are capable of delivering first data D1 to the control means 12 by appropriate connection, by wire, wirelessly or by other suitably data connection.

The control means 12 CON is also capable of operating the ventilation means by providing ventilator assistance so that said patient 5 is breathing spontaneously and/or breathing fully controlled by the ventilating means 11. As schematically indicated, a clinician, or other medical personnel, may survey and ultimately control the ventilation system 10. The control means is adapted for using both the first data D1, and second data D2 indicative of the respiratory feedback in the blood provided by measurement means 11c, in one or more physiological models MOD descriptive of, at least, lung mechanics, and/or gas exchange in the lungs of the patient, the physiological models comprising a number of model parameters MOD_P.

In one example of the invention, second data may particularly be described as data originating from other sources than the mechanical ventilator itself (this could be sensor, blood gases, doctor input etc.).

In one variant of the invention, the second data D2 could be estimated or guessed values being indicative of the respiratory feedback in the blood of said patient, preferably based on the medical history and/or present condition of the said patient. Thus, values from previously (earlier same day or previous days) could form the basis of such estimated guess for second data D2. In other variants, the second data can be provided from measurement means 11c on a continuous basis from actual measurements.

The control means is further arranged for simulating the effect on one, or more, model parameters MOD_P of the physiological models for a suggested value of the positive end expiratory pressure (PEEP) setting for the ventilation means, and thereby provide decision support in relation to said suggested PEEP value for the clinician. The suggested value of PEEP can be inputted by the clinician and/or suggested from the decision support part of the system 10 itself.

The effect on the model parameters MOD_P may be outputted to an appropriate human-machine interface (not shown) for displaying the result, e.g. a computer with a screen therefore. Alternatively or additionally, the model parameters MOD_P output may be communicated to the decision support part of the system 10 for use in connection with mechanical ventilation of patients, optionally for diagnostic purposes.

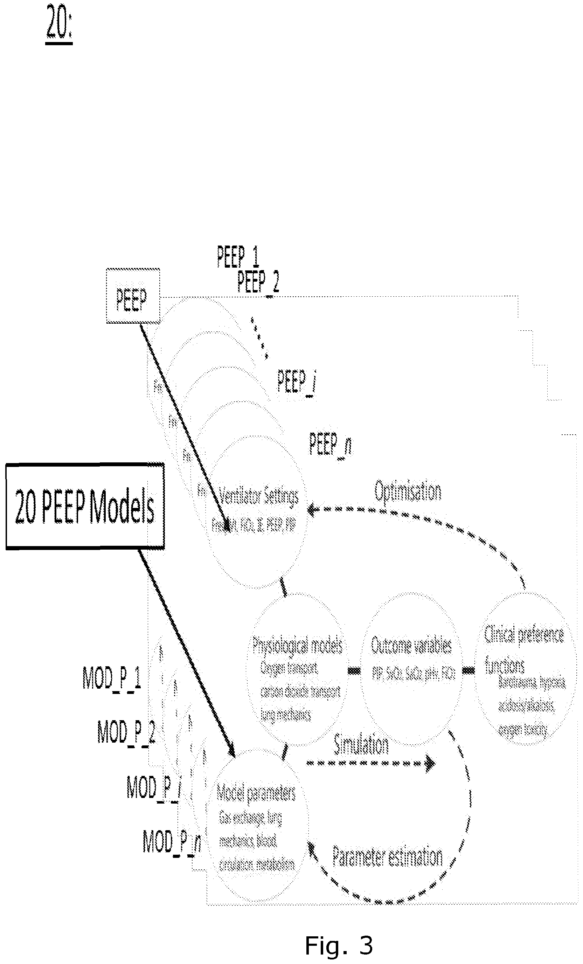

Figure 3 is a schematic drawing of the decision support system 20 for providing decision support to an associated mechanical ventilation system (not shown). PEEP models, cf. figure 2, reference 20, describe the expected and/or measured effect of PEEP on physiological model parameters MOD_P, these physiological models parameters may include parameters of gas exchange (such as shunt, V/Q mismatch, compliance), haemodynamics, metabolism etc. These PEEP models 20 then allow use of physiological models to simulate the effect of PEEP on the effect of changes in other mechanical ventilator settings by input to models of for example gas exchange, lung mechanics, blood acid-base, circulation and metabolism. Then the optimal setting of remaining ventilator settings can be found through model simulation of clinical outcome variables and optimisation of settings according to for example a set of clinical preference functions (CPFs), or a rule set. The present invention allows any implementation of model based decision support (the so-called decision support core in figure 2) to be applied, the example being a fully model based approach with models of relevant physiological systems and models of clinical preferences. By simulating the effect of PEEP on model parameters MOD_P, indicated as MOD_P_1, MOD_P_2, ....MOD_P_*i*, ...MOD_P_*n*, and further simulating how changes in model parameters affect optimal values of other ventilator settings in a hierarchical manner, it is possible to simulate for all plausible values of PEEP, indicated as PEEP_1, PEEP_2, ....PEEP_*i*, ...PEEP_*n*, either iteratively going through all possible values or by a numerical method searching through the PEEP values, thus locating the optimal PEEP value which can be advised to the clinician. The decision support core is therefore applied in several layers, each layer representing one PEEP value (see figure 3).

It may be noted that one PEEP value maybe have more than one model parameter value MOD_P associated to it, and only for illustrative purposes, a one-to-one relation between these two is shown in figure 3.

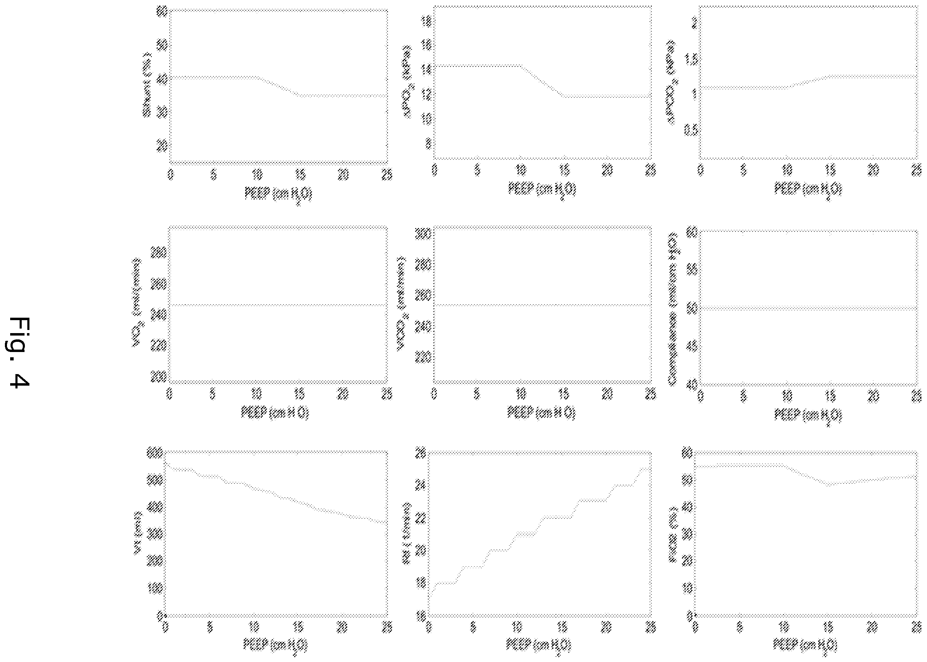

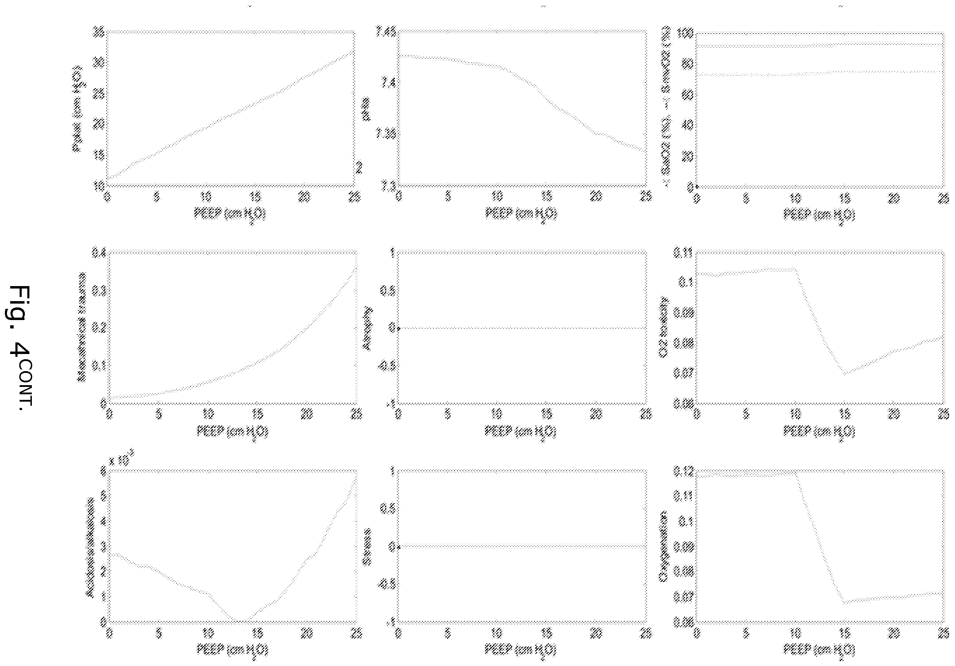

PEEP models can for example be of linear form with only expected changes in physiological parameters over a certain range of PEEP (see patient example with expected changes in shunt, deltaPO2, deltaPCO2, row 1, figure 4). The a priori expectation can also be that parameters remain constant with PEEP (see patient example with expected changes in VO2, VCO2, compliance, row 2, figure 4). With prediction of the effect of PEEP on physiological parameters, said parameters can then for a given value of PEEP be used in physiological models to predict changes in clinical outcome variables such that the outcome for a given set of mechanical ventilator setting may be evaluated in relation to clinical preferences. Then it is possible to obtain the optimal balance of competing clinical goals such as normalising patient acid-base status but preventing deleterious high settings of volume and pressures. As an example in data from a mechanically ventilated patient, figure 4 row 3 show changes in the optimal level of mechanical ventilator settings (tidal volume, respiratory frequency and inspired oxygen fraction) for different values of PEEP using the models of PEEP effect shown the top two rows. It can be seen that an increase in PEEP leads to reduction in tidal volume (Vt) which is necessary to prevent lung injury from high pressures. This is due to the compliance, which is predicted to stay constant leading to increases in ventilator pressures with PEEP. To counter the reduction in ventilation with tidal volume respiratory frequency (Rf) is increased. Finally, inspired oxygen level (FiO2) is reduced due to the predicted improvements in Shunt and ΔPO2. The fourth row of figure 4 shows predicted changes in clinical outcome variables with changes in PEEP. Despite tidal volume is reduced, the clinical outcome variable, plateau pressure (Pplat) increases with large changes in PEEP whilst pH and oxygenation (SaO2 and SmvO2) relatively constant, in a clinical perspective. The last two rows of figure 4 show examples of clinical preference functions representing different clinical goals and their predicted change with PEEP, which can be used for providing advice on the optimal level of PEEP.

The decision support depend on the PEEP selection strategy, as the effect of PEEP is different when set as part of an iterative set of increments in PEEP compared to an iterative set of decrements in PEEP following a recruitment manoeuvre. This is due to the hysteresis of pressure-volume relationship of human lungs. As PEEP is the ventilator maintaining a pressure during expiration during which the alveoli may collapse (de-recruit), PEEP is best set during expiration after a large increase of pressure to maintain open recently recruited alveoli as smaller levels of pressure are necessary to keep these lung units open (hysteresis). However, as recruitment maneuvers can be detrimental in some patients and the identification of whom may benefit from a recruitment manoeuvre is difficult, this approach to setting PEEP is primarily performed by clinical experts. Whilst these experts do perform recruitment maneuvers, little evidence supports an overall successful strategy. Therefore, at most institutions, PEEP changes are performed in small steps without first performing a recruitment maneuver, with the benefit of PEEP here being represented by the recruitment of alveoli due to small increases in pressure during inspiration and the PEEP maintaining these lung units open. As a consequence of the hysteresis of the lung pressure-volume relationship the optimal PEEP found during an incremental ("crawl") PEEP strategy is not the same as would have been found during a decremental (recruitment) strategy. The present invention therefore includes separate flows for setting PEEP depending on the PEEP selection strategy (see figure 6). In one example the system will have two sets of PEEP models representing each PEEP selection strategy, in the case where clinicians apply both strategies in the same patient. The clinician may chose the strategy via one, or more, PEEP selection strategy modules 30, cf. Figure 2. It may be advantageous if the system can provide advice on the optimal PEEP selection strategy based on the potential for lung recruitment in the patient, which may be assessed from the current values of the model parameters, the expected gain in these values from increases in pressure, and/or the measured changes in model parameters as a response to a change in pressure. For example, if a change in PEEP during a crawl strategy causes a large reduction in shunt indicating recruitment of alveoli, this may indicate large benefit from a recruitment maneuver and a shift in strategy. Also large levels of shunt per se, may indicate possible benefit from a recruitment maneuver. Based on the strategy selected by the clinician and/or the system and calculated decision support, the clinician or the system modifies the PEEP setting and other settings on the ventilator.

Changes in patient physiological variables are measured by measurement means for assessing ventilation, flow, pressure and volumes, and/or inspired and expired contents of O2 and CO2, and/or blood gas contents (O2 and CO2) and/or changes in acid base status of blood. These measurements are collected and processed. In a preferred embodiment, the data processing evaluates the changes in measurements and delay model parameter estimation until steady state is detected, this allowing the change in PEEP to take effect and avoiding the system from providing new advice when the previous advice has not had an effect.

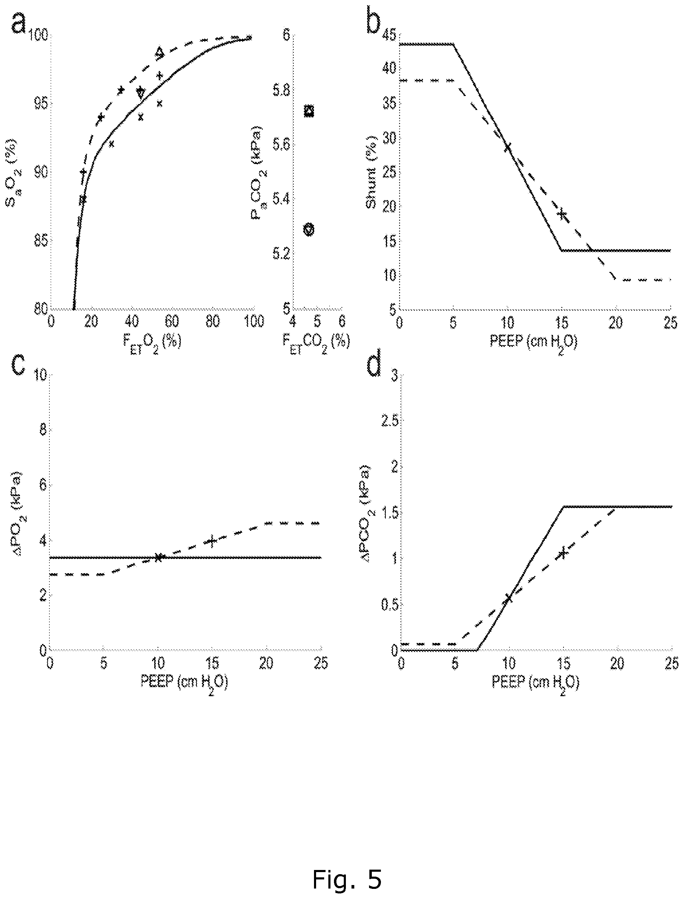

When the patient is in steady state or the system has in other ways evaluated that the PEEP has had an effect, the changes in patient physiological parameters can be measured. These may be available from a single point measurement, such as parameters for cardiac output, lung compliance, lung resistance, respiratory drive, anatomical dead space, alveolar deadspace, Effective shunt etc. However, some physiological parameters require the clinician to perturb the patient physiology for accurate measurement. For example, gas exchange parameters (e.g. shunt, low V/Q, high V/Q, dPO2, dPCO2, End expiratory lung volume) can be accurately measured from a stepwise variation in inspired oxygen fraction and measurement of several variables at steady state, cf. WO 2000 45702 and WO 2012069051, these two references describing the so-called automatic lung parameter estimator ('ALPE') of the present applicant, without and with carbon dioxide measurements, respectively. An example is shown in Figure 5a, where downward triangles and x's are measurements at low PEEP and upward triangles and +'s are measurements at a high PEEP value. The fit of a physiological model of gas exchange is shown by solid line for oxygen and o for carbon dioxide (right and plot), and a dashed line for oxygen and a square for carbon dioxide at low and high PEEP, respectively. It can be seen that the patient response to PEEP is an improvement in oxygenation shown by the leftwards shift of the FetO2-SaO2 curve and a worsening in carbon dioxide excretion, as caused by increase in high V/Q lung regions), seen as larger value of arterial partial pressure of carbon dioxide (PaCO2).

The estimated physiological model parameters are input to the PEEP model learning component where the PEEP models are adapted to the patient response to PEEP. Figure 5 shows an example for the PEEP learning models for gas exchange model parameters, and their adaptation to a measured response to a PEEP change. The PEEP models can be implemented as linear models. However, as the patient response is unknown a change in PEEP is preferably only assumed within a certain range beyond which model parameters are assumed to stay constant thus avoiding the decision support core to calculate too large changes in response to PEEP. This range can reflect the uncertainty in the assumed response and may be modified according to local clinical preferences and patient populations. When the models are updated so are these ranges reflecting the greater certainty regarding patient response to PEEP. To provide the first suggestion it is necessary to have an a priori expected response to PEEP. This may be patient population specific reflecting the different tendencies for specific patient populations to respond positively to PEEP. The model can be based on data as shown in figure 1. In figure 5 the a priori assumption is that shunt decreases with PEEP, low V/Q measured as dPO2 remains constant and high V/Q measured as dPCO2 increases. The patient responds somewhat differently which the models are adapted to, and these updated models may then be used by the decision support core to calculate a new optimal value of PEEP.

In addition to gas exchange, PEEP affects lung mechanics and in support ventilation modes PEEP may also affect metabolism. Lung mechanics are affected differently whether the patient is ventilated in a controlled ventilation mode where the ventilator fully initiates and carries out the breath or in support mode ventilation where the patient initiates the breaths and the ventilator supports the work of breathing. Figure 6 shows an example of a PEEP learning model for compliance for mechanical ventilation in control mode where it is not necessary to take the patient's own effort into account. In control mode, PEEP would theoretically affect compliance differently whether PEEP is increased or decreased due to the hysteresis of the lung pressure-volume relationship. In a preferred embodiment, the compliance model for control mode ventilation therefore has a steeper slope for simulating a decrease in PEEP reflecting the risk of causing a lung derecruitment where lung units collapse.

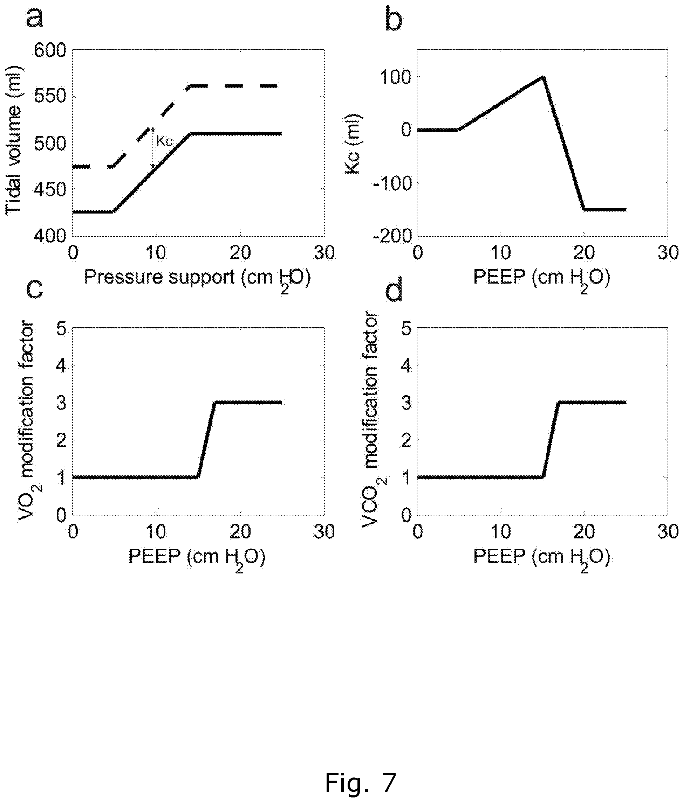

Figure 7a-b shows an example of a PEEP learning model for compliance for mechanical ventilation in support mode where it is necessary to take the patient's own effort into account. In support mode PEEP affects both lung mechanics and the respiratory work, lung mechanics by recruiting lung units, respiratory work by affecting the length tension relationship of the respiratory muscles thereby increasing/decreasing respiratory work. It is not possible from normally available bedside measurements to separate patient effort from lung mechanics. An example is therefore a combined model of effective lung compliance encompassing changes in patient respiratory work and lung mechanics with PEEP. Figure 7a shows a model of pressure-volume relationship in pressure support. The example models PEEP as affecting this relationship through the parameter kc shown in figure 7b where changes in kc displaces the PS-Vt model. As such if increases in PEEP worsens the length tension relationship of respiratory muscles kc decreases resulting in lower tidal volume for the same pressure support.

In support mode, PEEP may also affect patient metabolism as too high PEEP levels force the patient into a rapid shallow breathing pattern where respiratory work is significantly increased. To prevent the decision support core from advising on such PEEP levels if this response is observed, an example includes models of patient metabolism increasing several fold at this PEEP level. Figure 7c and 7d illustrate examples of these metabolism learning models, with a factor which is multiplied with oxygen consumption (VO2) or carbon dioxide production (VCO2) and is 1 for PEEP levels not causing a significant metabolic load.

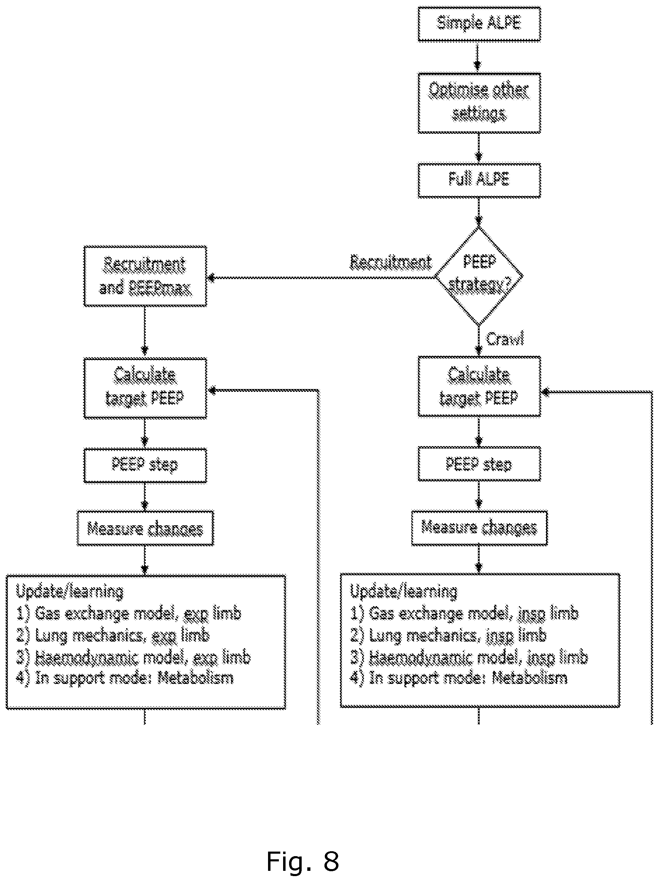

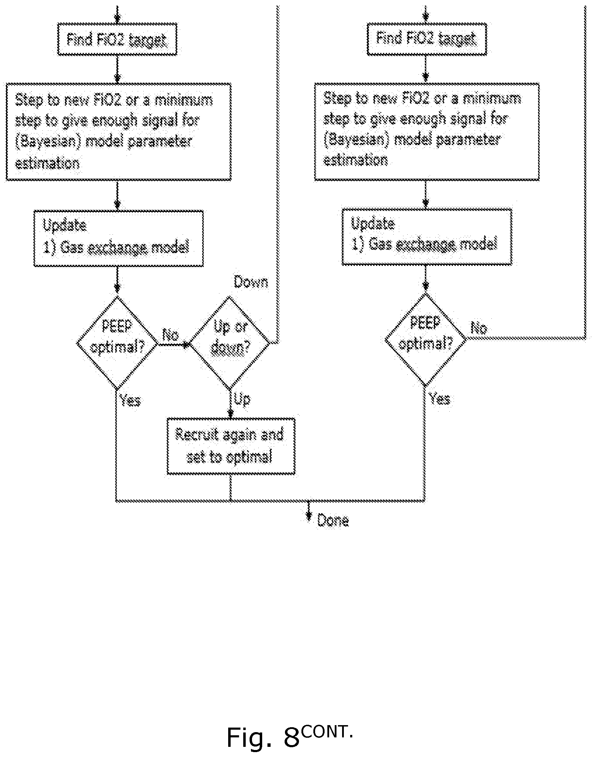

Figure 8 illustrates the algorithm flow for an example of the invention. In an example, the mechanical ventilation system first makes sure that gas exchange model parameters (simple 'ALPE' with no carbon dioxide and full 'ALPE' with carbon dioxide) are available and that the patient is stable with other ventilator settings set according to the decision support core. The flow is similar for the two possible PEEP strategies, with the most important difference being that learning models are describing the inspiratory limb and expiratory limb of the lung pressure-volume relationship for the crawl and the recruitment strategies, respectively. For each PEEP step, a target optimal PEEP is calculated, and a step in PEEP is then advised this step being the full PEEP step or a substep hereof, this substep being taken so as to minimize risk of adverse effects not known by the learning models at the current PEEP level. After each PEEP step, relevant learning models are updated where after one or several steps in FiO2 are calculated to learn the PEEP step effect on gas exchange and/or lung volumes. In an example the gas exchange models are updated in two steps, first following the initial step in PEEP, with this step giving a rough estimate of the gas exchange model parameters, these values being used for selecting the FiO2 target for subsequent steps.

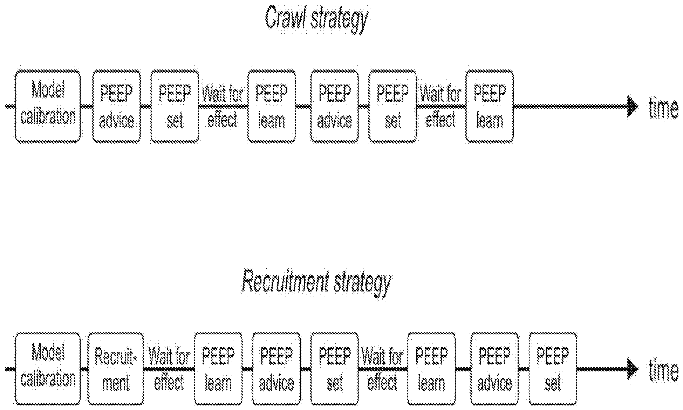

Figure 9 illustrates the timing of two PEEP steps for each PEEP selection strategy according to the flow depicted in figure 8. For the crawl strategy, an initial model calibration is performed, followed by a PEEP advice. This advice is then, if accepted by the clinician, set on the ventilator. After some time when the system signal processing indicates steady state the effect of PEEP is learned and a new PEEP advice is calculated which the clinician again shall review and if it is accepted it is set on the ventilator, and so follows until the system advices that the current PEEP is optimal or the clinician decides otherwise. As a difference, in the recruitment strategy, a recruitment manoeuvre is performed by the clinician after model calibration, where after a new maximum PEEP value is the starting point and the effect of the recruitment at this PEEP level is learned before the first PEEP step is advised to the clinician.

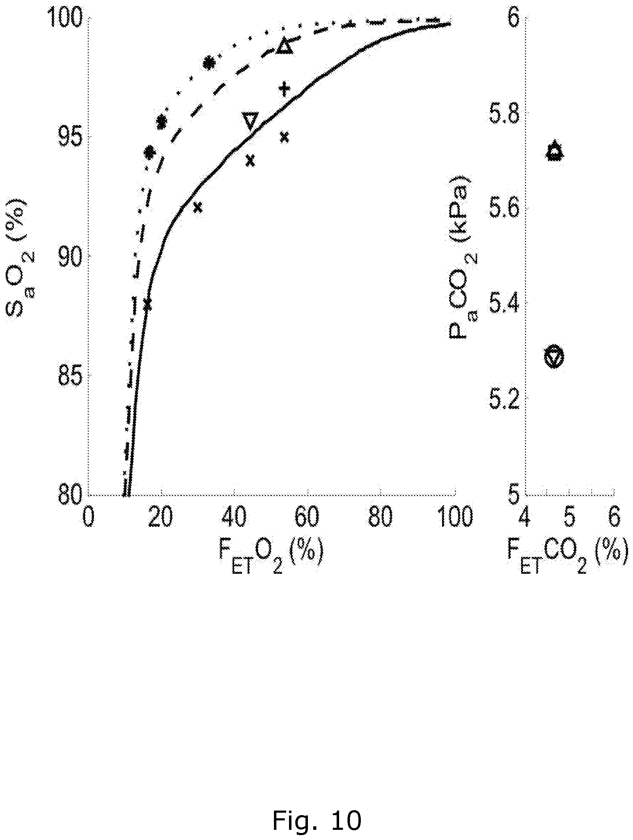

The procedure for measurement of gas exchange parameters shown in figure 5a requires 3 or more measurement points for best accuracy which is time-consuming, in particular if it must be performed repeatedly, once at each PEEP level during an optimization of PEEP. An example therefor uses fewer steps in FiO2 to learn changes in gas exchange parameters using both the expected effect and the measured effect using an approach weighing the error for both to learn as much as possible from the measurements and where the range of measurements are limited using the expected effect. Figure 10 shows a full gas exchange measurement at low PEEP (downwards triangle and x's for SaO2 measurements and downward triangle for PaCO2 measurement, solid line and o for model fit). Dotted line is expected response to PEEP increase with the three * being points selected as expected measurements in a range not measured with the upward triangle and + being the measured response. The dashed line illustrates a possible model fit utilising a weighted error based on fit to both expected and measured response. Any number of measured and/or expected points may be applied using either weighted/non-weighted error. If using few measurements, limitations in ranges of parameters can be used to allow a single solution. These ranges can be set so as to be conservative selecting the ranges that would require the highest levels of ventilator settings according to the decision support core so at so secure that if model parameters are inaccurate error is on the safe side.

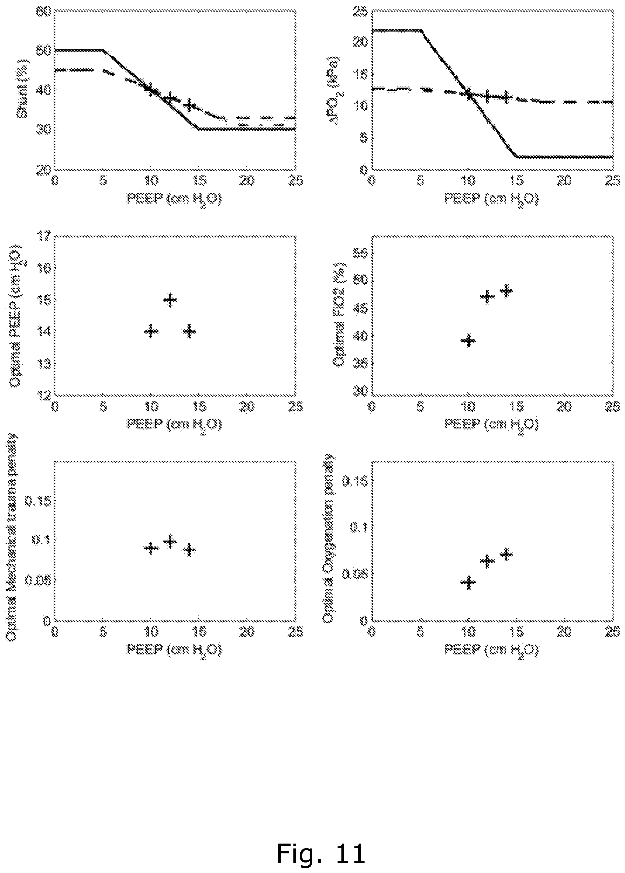

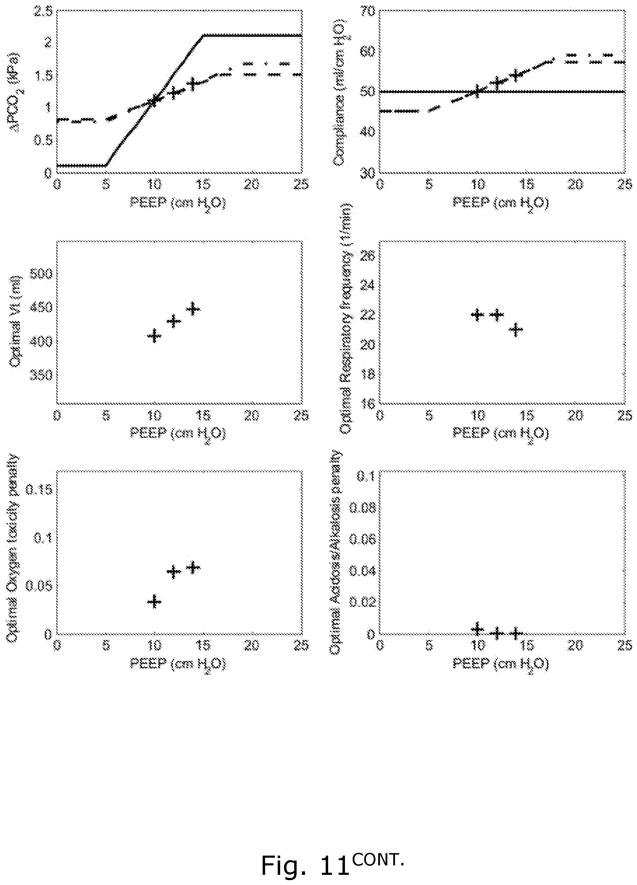

Figure 11 shows a simulated patient case for a patient in a control ventilator mode where an example of the invention is applied to select PEEP over three steps using the crawl PEEP selection strategy. The top row shows four of the PEEP learning models, one for shunt, low V/Q (dPO2), high V/Q (dPCO2) and lung mechanics (compliance). Solid line is the default expected response at baseline PEEP value, dashed line is the updated learning model after first step, with dash-dot being the final update of the PEEP learning models before the decision support core advices that current PEEP is the optimal level. Second row shows the optimal value of PEEP and other ventilator settings according to patient response to PEEP and the final row shows changes in mathematical clinical preference functions from a decision support core reflecting the optimal ventilator settings. Steps are not necessarily performed at full to the target, with smaller steps allowing the patient response to be learned in a safe manner.

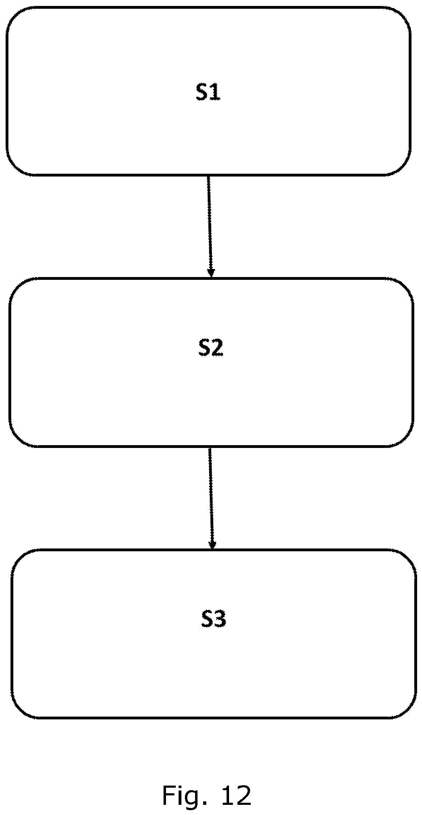

Figure 12 is a schematic system-chart representing an out-line of/in detail the operations of the method, or computer program product according to the invention, i.e. a method for operating an mechanical ventilation system 10 for respiration of an associated patient 5, as shown in figure 2, the system being adapted for providing decision support for mechanical ventilation, the method comprising:
- **S1** providing ventilation means 11 capable of mechanical ventilating said patient with air and/or one or more medical gases, the ventilation means having a plurality of settings (V_SET) comprising a positive end expiratory pressure (PEEP) setting,
- **S2** providing control means 12, the ventilator means being controllable by said control means by operational connection thereto, and
- **S3** providing measurement means, 11a and 11b, arranged for measuring the inspired gas and/or the respiratory feedback of said patient in the expired gas in response to the mechanical ventilation, the measurement means being capable of delivering first data D1 to said control means,
wherein the control means is adapted for using both the first data D1 and second data D2 indicative of the respiratory feedback in the blood in physiological models, MOD, descriptive of, at least, lung mechanics, and/or gas exchange in the lungs of the patient, the physiological models comprising a number of model parameters, MOD_P, and
wherein the control means is further arranged for simulating the effect on one, or more, model parameters, MOD_P, of the physiological models for a suggested value of the positive end expiratory pressure (PEEP) setting for the ventilation means, and thereby provide decision support in relation to said suggested PEEP value.

The invention can be implemented by means of hardware, software, firmware or any combination of these. The invention or some of the features thereof can also be implemented as software running on one or more data processors and/or digital signal processors.

The individual elements of an example of the invention may be physically, functionally and logically implemented in any suitable way such as in a single unit, in a plurality of units or as part of separate functional units. The invention may be implemented in a single unit, or be both physically and functionally distributed between different units and processors.

In short, the invention relates to a mechanical ventilation system 10 for respiration of a patient 5, the system being adapted for providing decision support for mechanical ventilation. Control means 12 is adapted for using both first data D1 and second data D2, indicative of the respiratory feedback in the blood, in physiological models MOD descriptive of, at least, lung mechanics, and/or gas exchange in the lungs of the patient, the physiological models comprising a number of model parameters MOD_P. The control means is further arranged for simulating the effect on one, or more, model parameters MOD_P of the physiological models for a suggested value of the positive end expiratory pressure (PEEP) setting for the ventilation means, and thereby provide decision support in relation to said suggested PEEP value. The invention is advantageous for providing mathematical based models of changes in physiology in response to changes in ventilator settings of the PEEP thereby allowing mathematical physiological models to predict changes in clinical variables for a given value of PEEP.

### Glossary

- Vt: Respiratory volume in a single breath, tidal volume
- RR: respiratory frequency (RR) or, equivalently, duration of breath (including duration of inspiratory or expiratory phase)
- pHa: Arterial blood pH
- PaCO2: Pressure of carbon dioxide in arterial blood,
- SaO2: Oxygen saturation of arterial blood
- PaO2: Pressure of oxygen in arterial blood

### References

1. The Acute Respiratory Distress Syndrome (ARDS) Network (2000) Ventilation with lower tidal volumes as compared with traditional tidal volumes for acute lung injury and the acute respiratory distress syndrome. N Engl. J Med. 342:1301-1308.
2. Rees SE, Allerød C, Murley D, Zhao Y, Smith BW, Kjærgaard S, Thorgaard p, Andreassen S (2006) Using physiological models and decision theory for selecting appropriate ventilator settings. J Clin Monit Comput. 20:421-429.
3. Karbing DS, Allerød C, Thomsen LP, Espersen K, Thorgaard P, Andreassen S, Kjærgaard S, Rees SE (2012) Retrospective evaluation of a decision supports system for controlled mechanical ventilation. Med Biol Eng Comput. 50:43-51.
4. Kwok HF, Linkens DA, Mahfouf M, Mills GH (2004). SIVA: A Hybrid Knowledge- and-Model-Based Advisory System for Intensive Care Ventilators. IEEE TRANSACTIONS ON INFORMATION TECHNOLOGY IN BIOMEDICINE. 8:161-172.
5. Tehrani DF and Roum JH (2008). Intelligent decision support systems for mechanical ventilation. Artificial Intelligence in Medicine. 44:171-182.
6. Mélot C. Contribution of multiple inert gas elimination technique to pulmonary medicine - 5 (1994). Thorax. 49:1251-1258.
7. Murley D, Rees S, Rasmussen B, Andreassen S (2005). Decision support of inspired oxygen selection based on Bayesian learning of pulmonary gas exchange parameters. Artificial Intelligence in Medicine. 34:53-63.
8. Rees SE and Andreassen S. Mathematical models of oxygen and carbon dioxide storage and transport: the acid base chemistry of blood (2005). Crit Rev Biomed Eng. 33:209-264.
9. Otis AB, Fenn WO, Rahn H. Mechanics of breathing in man (1950). J Appl Phys. 2: 592-607.

## Claims

1. A mechanical ventilation system (10) for respiration of an associated patient (5), the system being adapted for providing decision support for mechanical ventilation, the system comprising:
- ventilation means (11) capable of mechanical ventilating said patient (5) with air and/or one or more medical gases, the ventilation means (11) having a plurality of settings (V_SET) comprising a positive end expiratory pressure (PEEP) setting,
- control means (12), the ventilator means (11) being controllable by said control means (12) by operational connection thereto, and
- measurement means (11a, 11b) arranged for measuring the inspired gas and/or the respiratory feedback of said patient (5) in the expired gas in response to the mechanical ventilation, the measurement means (11a, 11b) being capable of delivering first data (D1) to said control means (12),
wherein the control means (12) is adapted for using both the first data (D1) and second data (D2) indicative of the respiratory feedback in the blood in physiological models (MOD) descriptive of, at least, lung mechanics, and/or gas exchange in the lungs of the patient, the physiological models comprising a number of model parameters (MOD_P), and
**characterized in that**
the control means (12) is further arranged for simulating the effect on one, or more, model parameters (MOD_P) selected from lung mechanics parameters, gas exchange parameters, blood-acid-base parameters, respiratory drive parameters and circulation parameters of the physiological models for a suggested value of the positive end expiratory pressure (PEEP) setting for the ventilation means (11), and thereby provide decision support in relation to said suggested PEEP value.

2. The mechanical ventilation system (10) according to claim 1, wherein the control means (12) is further arranged for simulating the effect on one, or more, model parameters (MOD_P) of the physiological models for a plurality of values (PEEP;1,...,n) of the positive end expiratory pressure setting for the ventilation means (11), and thereby provide decision support in relation to said plurality of PEEP values.

3. The mechanical ventilation system (10) according to claim 2, wherein the control means (12) is further arranged for suggesting an optimum value between the plurality of values of the positive end expiratory pressure setting for the ventilation means (PEEP;1,...,n).

4. The mechanical ventilation system (10) according to claim 1 or 2, wherein the control means (12) is further arranged for simulating the effect on one, or more, parameters (MOD_P) of the physiological models for one, or more, values in the positive end expiratory pressure setting for the ventilation means (PEEP) performed by a simulation based on at least two previous values of the PEEP setting for the ventilation means (11).

5. The mechanical ventilation system according to claim 1 or 2, wherein the control means (12) is further arranged for simulating the effect on one, or more, parameters (MOD_P) of the physiological models for one, or more, values in the positive end expiratory pressure setting for the ventilation means (PEEP) performed by a simulation based on at least two simulated values of the PEEP setting for the ventilation means (11).

6. The mechanical ventilation system (10) according to claim 1, wherein the control means (12) comprises one, or more, positive end expiratory pressure (PEEP) models (20), each PEEP model comprising a model parameter (MOD_P) of a physiological model as a function of the PEEP settings for the ventilation means (11).

7. The mechanical ventilation system (10) according to claim 6, wherein one, or more, of the PEEP models (20) are adapted to the patient, and/or the clinical condition of the patient, before initiating changes of the PEEP settings, and/or while changing the PEEP settings.

8. The mechanical ventilation system (10) according to claim 7, wherein the one, or more, PEEP models (20) are adapted to the patient by a learning module, the learning module having a set of a priori settings of PEEP model parameters (MOD_P_PEEP) based on the currently measured model parameter (MOD_P) value of the corresponding physiological models (MOD), preferably based on a Bayesian distribution based on patient type and/or clinical condition.

9. The mechanical ventilation system (10) according to claim 1, wherein one, or more, of the physiological models (MOD) are adapted to the patient, and/or the clinical condition of the patient, before initiating changes of the PEEP settings, and/or while changing the PEEP settings.

10. The mechanical ventilation system (10) according to claim 1 or 9, wherein one, or more, additional physiological models (MOD) are further descriptive of the metabolism of the patient, the blood circulation of the patient, acid-base status of the patient, oxygen and/or carbon dioxide transport for the patient, and/or the respiratory drive of the patient.

11. The mechanical ventilation system (10) according to claim 10, wherein at least two physiological models are integrated by having one, or more, variables in common.

12. The mechanical ventilation system (10) according to claim 1, wherein the control means (12) comprises one, or more, modules (30) for choosing a PEEP changing strategy for the patient (5).

13. The mechanical ventilation system (10) according to claim 12, wherein one choice of PEEP changing strategy is based on an assumption of lung recruitment wherein a relatively high airway pressure is applied for a relatively short time followed by stepwise PEEP reduction until the optimal balance is reached.

14. The mechanical ventilation system (10) according to claim 12 or 13, wherein one choice of PEEP changing strategy is based on a stepwise increase of PEEP until an optimal balance is reached.

15. The mechanical ventilation system (10) according to claim 1, wherein the control means (12) further comprises a plurality of clinical preference functions (CPFs) relating settings of positive end expiratory pressure (PEEP) for the ventilation means to a corresponding set of clinical outcome variables.

16. The mechanical ventilation system (10) according to any of the preceding claims, wherein the positive end expiratory pressure (PEEP) setting is further optimized with respect to other mechanical ventilation settings, preferably inspired oxygen (FIO2), tidal volume (VT), pressure above PEEP, and/or respiratory frequency.

17. A method for operating an mechanical ventilation system (10) for respiration of an associated patient (5), the system being adapted for providing decision support for mechanical ventilation, the method comprising:
- providing ventilation means (11) capable of mechanical ventilating said patient (5) with air and/or one or more medical gases, the ventilation means (11) having a plurality of settings (V_SET) comprising a positive end expiratory pressure (PEEP) setting,
- providing control means (12), the ventilator means (11) being controllable by said control (12) means by operational connection thereto, and
- providing measurement means (11a, 11b) arranged for measuring the inspired gas and/or the respiratory feedback of said patient (5) in the expired gas in response to the mechanical ventilation, the measurement means (11a, 11b) being capable of delivering first data (D1) to said control means (12),
wherein the control means (12) is adapted for using both the first data (D1) and second data (D2) indicative of the respiratory feedback in the blood in physiological models (MOD) descriptive of, at least, lung mechanics, and/or gas exchange in the lungs of the patient, the physiological models comprising a number of model parameters (MOD_P), and
**characterized in that**
the control means is further arranged for simulating the effect on one, or more, model parameters (MOD_P) selected from lung mechanics parameters, gas exchange parameters, blood-acid-base parameters, respiratory drive parameters and circulation parameters of the physiological models for a suggested value of the positive end expiratory pressure (PEEP) setting for the ventilation means (11), and thereby provide decision support in relation to said suggested PEEP value.

18. A computer program product being adapted to enable a computer system comprising at least one computer having data storage means in connection therewith to control a mechanical ventilation system according to the method in claim 17.

## Patentansprüche

1. Mechanisches Belüftungssystem (10) zur Beatmung eines verbundenen Patienten (5), wobei das System ausgelegt ist, um Entscheidungsunterstützung für die mechanische Belüftung zu bieten, wobei das System Folgendes umfasst:
- eine Belüftungseinrichtung (11), die in der Lage ist, den Patienten (5) mechanisch mit Luft und/oder einem oder mehreren medizinischen Gasen zu belüften, wobei die Belüftungseinrichtung (11) eine Vielzahl von Einstellungen (V_SET) aufweist, die eine Einstellung des positiven endexpiratorischen Drucks (PEEP) umfasst,
- eine Steuerungseinrichtung (12), wobei die Belüftungseinrichtung (11) durch die Steuerungseinrichtung (12) durch eine Wirkverbindung dazu gesteuert werden kann, und
- eine Messeinrichtung (11a, 11b), die zum Messen des Einatmungsgases und/oder der Atemrückmeldung des Patienten (5) in dem Ausatmungsgas als Reaktion auf die mechanische Belüftung angeordnet ist, wobei die Messeinrichtung (11a, 11b) in der Lage ist, erste Daten (D1) an die Steuerungseinrichtung (12) zu liefern,
wobei die Steuerungseinrichtung (12) ausgelegt ist, um sowohl die ersten Daten (D1) als auch zweite Daten (D2), die die Atemrückmeldung im Blut in physiologischen Modellen (MOD) angeben, die mindestens die Lungenmechanik und/oder den Gasaustausch in den Lungen des Patienten beschreiben, zu verwenden, wobei die physiologischen Modelle eine Anzahl von Modellparametern (MOD_P) umfassen, und
**dadurch gekennzeichnet, dass**
die Steuerungseinrichtung (12) ferner angeordnet ist, um die Wirkung auf einen oder mehrere Modellparameter (MOD_P), der/die aus den Lungenmechanikparametern, Gasaustauschparametern, Blut-Säure-Base-Parametern, Atemantriebsparametern und Kreislaufparametern der physiologischen Modelle ausgewählt ist/sind, für einen vorgeschlagenen Wert der Einstellung des positiven endexpiratorischen Drucks (PEEP) für die Belüftungseinrichtung (11) zu simulieren und dadurch Entscheidungsunterstützung in Bezug auf den vorgeschlagenen PEEP-Wert zu bieten.

2. Mechanisches Belüftungssystem (10) nach Anspruch 1, wobei die Steuerungseinrichtung (12) ferner angeordnet ist, um die Wirkung auf einen oder mehrere Modellparameter (MOD_P) der physiologischen Modelle für eine Vielzahl von Werten (PEEP; 1,...,n) der Einstellung des positiven endexpiratorischen Drucks für die Belüftungseinrichtung (11) zu simulieren und dadurch Entscheidungsunterstützung in Bezug auf die Vielzahl von PEEP-Werten zu bieten.

3. Mechanisches Belüftungssystem (10) nach Anspruch 2, wobei die Steuerungseinrichtung (12) ferner angeordnet ist, um einen optimalen Wert unter der Vielzahl von Werten der Einstellung des positiven endexpiratorischen Drucks für die Belüftungseinrichtung (PEEP; 1,...,n) vorzuschlagen.

4. Mechanisches Belüftungssystem (10) nach Anspruch 1 oder 2, wobei die Steuerungseinrichtung (12) ferner angeordnet ist, um die Wirkung auf einen oder mehrere Parameter (MOD_P) der physiologischen Modelle für einen oder mehrere Werte in der Einstellung des positiven endexpiratorischen Drucks für die Belüftungseinrichtung (PEEP) zu simulieren, was durch eine Simulation basierend auf mindestens zwei vorherigen Werten der PEEP-Einstellung für die Belüftungseinrichtung (11) durchgeführt wird.

5. Mechanisches Belüftungssystem nach Anspruch 1 oder 2, wobei die Steuerungseinrichtung (12) ferner angeordnet ist, um die Wirkung auf einen oder mehrere Parameter (MOD_P) der physiologischen Modelle für einen oder mehrere Werte in der Einstellung des positiven endexpiratorischen Drucks für die Belüftungseinrichtung (PEEP) zu simulieren, was durch eine Simulation basierend auf mindestens zwei simulierten Werten der PEEP-Einstellung für die Belüftungseinrichtung (11) durchgeführt wird.

6. Mechanisches Belüftungssystem (10) nach Anspruch 1, wobei die Steuerungseinrichtung (12) ein oder mehrere Modelle für den positiven endexpiratorischen Druck (PEEP) (20) umfasst, wobei jedes PEEP-Modell einen Modellparameter (MOD_P) eines physiologischen Modells abhängig von den PEEP-Einstellungen für die Belüftungseinrichtung (11) umfasst.

7. Mechanisches Belüftungssystem (10) nach Anspruch 6, wobei eines oder mehrere der PEEP-Modelle (20) für den Patienten und/oder den klinischen Zustand des Patienten angepasst wird/werden, bevor Änderungen der PEEP-Einstellungen eingeleitet werden und/oder während die PEEP-Einstellungen geändert werden.

8. Mechanisches Belüftungssystem (10) nach Anspruch 7, wobei eines oder mehrere der PEEP-Modelle (20) für den Patienten durch ein Lernmodul angepasst wird/werden, wobei das Lernmodul einen Satz A-priori-Einstellungen von PEEP-Modellparametern (MOD_P_PEEP) basierend auf dem aktuell gemessenen Wert des Modellparameters (MOD_P) der entsprechenden physiologischen Modelle (MOD) aufweist, vorzugsweise basierend auf einer Bayes-Verteilung basierend auf dem Patiententyp und/oder dem klinischen Zustand.

9. Mechanisches Belüftungssystem (10) nach Anspruch 1, wobei eines oder mehrere der physiologischen Modelle (MOD) für den Patienten und/oder den klinischen Zustand des Patienten angepasst wird/werden, bevor Änderungen der PEEP-Einstellungen eingeleitet werden und/oder während die PEEP-Einstellungen geändert werden.

10. Mechanisches Belüftungssystem (10) nach Anspruch 1 oder 9, wobei eines oder mehrere zusätzliche physiologische Modelle (MOD) ferner den Metabolismus des Patienten, die Blutzirkulation des Patienten, den Säure-Base-Status des Patienten den Sauerstoff- und/oder Kohlendioxidtransport für den Patienten und/oder den Atemantrieb des Patienten beschreibt/beschreiben.

11. Mechanisches Belüftungssystem (10) nach Anspruch 10, wobei mindestens zwei physiologische Modelle integriert sind, indem sie eine oder mehrere Variablen gemeinsam haben.

12. Mechanisches Belüftungssystem (10) nach Anspruch 1, wobei die Steuerungseinrichtung (12) ein oder mehrere Module (30) zum Auswählen einer PEEP-Änderungsstrategie für den Patienten (5) umfasst.

13. Mechanisches Belüftungssystem (10) nach Anspruch 12, wobei eine Wahl der PEEP-Änderungsstrategie auf einer Annahme des Lungen-Recruitment basiert, wobei ein relativ hoher Atemwegsdruck für eine relativ kurze Zeit angewandt wird, gefolgt von schrittweiser PEEP-Reduzierung, bis das optimale Gleichgewicht erreicht wird.

14. Mechanisches Belüftungssystem (10) nach Anspruch 12 oder 13, wobei eine Wahl der PEEP-Änderungsstrategie auf einer schrittweisen Erhöhung des PEEP, bis ein optimales Gleichgewicht erreicht wird, basiert.

15. Mechanisches Belüftungssystem (10) nach Anspruch 1, wobei die Steuerungseinrichtung (12) ferner eine Vielzahl klinischer Präferenzfunktionen (CPFs) umfasst, die die Einstellungen des positiven endexpiratorischen Drucks (PEEP) für die Belüftungseinrichtung mit einem entsprechenden Satz klinischer Ergebnisvariablen in Verbindung bringen.

16. Mechanisches Belüftungssystem (10) nach einem der vorstehenden Ansprüche, wobei die Einstellung des positiven endexpiratorischen Drucks (PEEP) ferner in Bezug auf andere Einstellungen der mechanischen Belüftung optimiert wird, vorzugsweise inspiratorischer Sauerstoff (FIO2), Tidalvolumen (VT), Druck über PEEP und/oder Atemfrequenz.

17. Verfahren zum Betreiben eines mechanischen Belüftungssystems (10) zur Beatmung eines verbundenen Patienten (5), wobei das System ausgelegt ist, um Entscheidungsunterstützung für die mechanische Belüftung zu bieten, wobei das Verfahren Folgendes umfasst:
- Bereitstellen einer Belüftungseinrichtung (11), die in der Lage ist, den Patienten (5) mechanisch mit Luft und/oder einem oder mehreren medizinischen Gasen zu belüften, wobei die Belüftungseinrichtung (11) eine Vielzahl von Einstellungen (V_SET) aufweist, die eine Einstellung des positiven endexpiratorischen Drucks (PEEP) umfasst,
- Bereitstellen einer Steuerungseinrichtung (12), wobei die Belüftungseinrichtung (11) durch die Steuerungseinrichtung (12) durch eine Wirkverbindung dazu gesteuert werden kann, und
- Bereitstellen einer Messeinrichtung (11a, 11b), die zum Messen des Einatmungsgases und/oder der Atemrückmeldung des Patienten (5) in dem Ausatmungsgas als Reaktion auf die mechanische Belüftung angeordnet ist, wobei die Messeinrichtung (11a, 11b) in der Lage ist, erste Daten (D1) an die Steuerungseinrichtung (12) zu liefern,
wobei die Steuerungseinrichtung (12) ausgelegt ist, um sowohl die ersten Daten (D1) als auch zweite Daten (D2), die die Atemrückmeldung im Blut in physiologischen Modellen (MOD) angeben, die mindestens die Lungenmechanik und/oder den Gasaustausch in den Lungen des Patienten beschreiben, zu verwenden, wobei die physiologischen Modelle eine Anzahl von Modellparametern (MOD_P) umfassen, und
**dadurch gekennzeichnet, dass**
die Steuerungseinrichtung ferner angeordnet ist, um die Wirkung auf einen oder mehrere Modellparameter (MOD_P), der/die aus den Lungenmechanikparametern, Gasaustauschparametern, Blut-Säure-Base-Parametern, Atemantriebsparametern und Kreislaufparametern der physiologischen Modelle ausgewählt ist/sind, für einen vorgeschlagenen Wert der Einstellung des positiven endexpiratorischen Drucks (PEEP) für die Belüftungseinrichtung (11) zu simulieren und dadurch Entscheidungsunterstützung in Bezug auf den vorgeschlagenen PEEP-Wert zu bieten.

18. Computerprogrammprodukt, das ausgelegt ist, um es einem Computersystem, das mindestens einen Computer umfasst, der eine Datenspeichereinrichtung in Verbindung damit aufweist, zu ermöglichen, ein mechanisches Belüftungssystem gemäß dem Verfahren in Anspruch 17 zu steuern.

## Revendications

1. Système de ventilation mécanique (10) pour la respiration d'un patient associé (5), le système étant adapté pour fournir une aide à la décision pour une ventilation mécanique, le système comprenant :
- un moyen de ventilation (11) capable de ventiler mécaniquement ledit patient (5) avec de l'air et/ou un ou plusieurs gaz médicaux, le moyen de ventilation (11) ayant une pluralité de réglages (V_SET) comprenant un réglage de pression expiratoire positive (PEEP),
- un moyen de commande (12), le moyen ventilateur (11) pouvant être commandé par ledit moyen de commande (12) par un raccordement opérationnel à celui-ci, et
- un moyen de mesure (11a, 11b) agencé pour mesurer le gaz inspiré et/ou le retour respiratoire dudit patient (5) dans le gaz expiré en réponse à la ventilation mécanique, le moyen de mesure (11a, 11b) étant capable de délivrer des premières données (D1) audit moyen de commande (12),
dans lequel le moyen de commande (12) est adapté pour utiliser à la fois les premières données (D1) et des secondes données (D2) indicatives du retour respiratoire dans le sang dans des modèles physiologiques (MOD) descriptifs au moins d'une mécanique ventilatoire, et/ou d'un échange de gaz dans les poumons du patient, les modèles physiologiques comprenant un nombre de paramètres de modèle (MOD_P), et
**caractérisé en ce que**
le moyen de commande (12) est en outre agencé pour simuler l'effet sur un ou plusieurs paramètres de modèle (MOD_P) choisis parmi des paramètres de mécanique ventilatoire, des paramètres d'échange de gaz, des paramètres sang-acide-base, des paramètres de pulsion respiratoire et des paramètres de circulation des modèles physiologiques pour une valeur suggérée du réglage de pression expiratoire positive (PEEP) pour le moyen de ventilation (11), et fournir ainsi une aide à la décision par rapport à ladite valeur PEEP suggérée.

2. Système de ventilation mécanique (10) selon la revendication 1, dans lequel le moyen de commande (12) est en outre agencé pour simuler l'effet sur un ou plusieurs paramètres de modèle (MOD_P) des modèles physiologiques pour une pluralité de valeurs (PEEP ; 1, ..., n) du réglage de pression expiratoire positive pour le moyen de ventilation (11), et fournir ainsi une aide à la décision par rapport à ladite pluralité de valeurs PEEP.

3. Système de ventilation mécanique (10) selon la revendication 2, dans lequel le moyen de commande (12) est en outre agencé pour suggérer une valeur optimale entre la pluralité de valeurs du réglage de pression expiratoire positive pour le moyen de ventilation (PEEP ; 1, ..., n).

4. Système de ventilation mécanique (10) selon la revendication 1 ou 2, dans lequel le moyen de commande (12) est en outre agencé pour simuler l'effet sur un ou plusieurs paramètres (MOD_P) des modèles physiologiques pour une ou plusieurs valeurs dans le réglage de pression expiratoire positive (PEEP) pour le moyen de ventilation réalisé par une simulation d'après au moins deux valeurs précédentes du réglage PEEP pour le moyen de ventilation (11).

5. Système de ventilation mécanique selon la revendication 1 ou 2, dans lequel le moyen de commande (12) est en outre agencé pour simuler l'effet sur un ou plusieurs paramètres (MOD_P) des modèles physiologiques pour une ou plusieurs valeurs dans le réglage de pression expiratoire positive (PEEP) pour le moyen de ventilation réalisé par une simulation d'après au moins deux valeurs simulées du réglage PEEP pour le moyen de ventilation (11).

6. Système de ventilation mécanique (10) selon la revendication 1, dans lequel le moyen de commande (12) comprend un ou plusieurs modèles de pression expiratoire positive (PEEP) (20), chaque modèle PEEP comprenant un paramètre de modèle (MOD_P) d'un modèle physiologique en fonction des réglages PEEP pour le moyen de ventilation (11).

7. Système de ventilation mécanique (10) selon la revendication 6, dans lequel un ou plusieurs des modèles PEEP (20) sont adaptés au patient, et/ou à l'état clinique du patient, avant de provoquer des changements des réglages PEEP, et/ou tout en changeant les réglages PEEP.

8. Système de ventilation mécanique (10) selon la revendication 7, dans lequel les un ou plusieurs modèles PEEP (20) sont adaptés au patient par un module d'apprentissage, le module d'apprentissage ayant un ensemble de réglages a priori de paramètres de modèle PEEP (MOD_P_PEEP) d'après la valeur de paramètre de modèle (MOD_P) mesurée actuellement des modèles physiologiques (MOD) correspondants, de préférence d'après une distribution bayésienne basée sur un type et/ou un état clinique du patient.

9. Système de ventilation mécanique (10) selon la revendication 1, dans lequel un ou plusieurs des modèles physiologiques (MOD) sont adaptés au patient, et/ou à l'état clinique du patient, avant de provoquer des changements des réglages PEEP, et/ou tout en changeant les réglages PEEP.

10. Système de ventilation mécanique (10) selon la revendication 1 ou 9, dans lequel un ou plusieurs modèles physiologiques (MOD) supplémentaires sont en outre descriptifs du métabolisme du patient, de la circulation sanguine du patient, d'un statut acide-base du patient, d'un transport d'oxygène et/ou de dioxyde de carbone pour le patient, et/ou de la pulsion respiratoire du patient.

11. Système de ventilation mécanique (10) selon la revendication 10, dans lequel au moins deux modèles physiologiques sont intégrés en ayant une ou plusieurs variables en commun.

12. Système de ventilation mécanique (10) selon la revendication 1, dans lequel le moyen de commande (12) comprend un ou plusieurs modules (30) pour choisir une stratégie de changement PEEP pour le patient (5).

13. Système de ventilation mécanique (10) selon la revendication 12, dans lequel un choix de stratégie de changement PEEP est basé sur l'hypothèse d'un recrutement pulmonaire dans lequel une pression des voies aériennes relativement élevée est appliquée pendant un temps relativement court suivie d'une réduction PEEP par paliers jusqu'à ce que l'équilibre optimal soit atteint.

14. Système de ventilation mécanique (10) selon la revendication 12 ou 13, dans lequel, un choix de stratégie de changement PEEP est basé sur une augmentation par paliers de PEEP jusqu'à ce qu'un équilibre optimal soit atteint.

15. Système de ventilation mécanique (10) selon la revendication 1, dans lequel le moyen de commande (12) comprend en outre une pluralité de fonctions de préférence clinique (CPF) concernant des réglages de pression expiratoire positive (PEEP) pour le moyen de ventilation à un ensemble correspondant de variables de résultat clinique.

16. Système de ventilation mécanique (10) selon l'une quelconque des revendications précédentes, dans lequel le réglage de pression expiratoire positive (PEEP) est en outre optimisé par rapport à d'autres réglages de ventilation mécanique, de préférence l'oxygène inspiré (FIO2), le volume respiratoire (VT), la pression supérieure à PEEP, et/ou la fréquence respiratoire.

17. Procédé de fonctionnement d'un système de ventilation mécanique (10) pour la respiration d'un patient associé (5), le système étant adapté pour fournir une aide à la décision pour une ventilation mécanique, le procédé comprenant :
- la fourniture d'un moyen de ventilation (11) capable de ventiler mécaniquement ledit patient (5) avec de l'air et/ou un ou plusieurs gaz médicaux, le moyen de ventilation (11) ayant une pluralité de réglages (V_SET) comprenant un réglage de pression expiratoire positive (PEEP),
- la fourniture d'un moyen de commande (12), le moyen ventilateur (11) pouvant être commandé par ledit moyen de commande (12) par un raccordement opérationnel à celui-ci, et
- la fourniture d'un moyen de mesure (11a, 11b) agencé pour mesurer le gaz inspiré et/ou le retour respiratoire dudit patient (5) dans le gaz expiré en réponse à la ventilation mécanique, le moyen de mesure (11a, 11b) étant capable de délivrer des premières données (D1) audit moyen de commande (12), dans lequel le moyen de commande (12) est adapté pour utiliser à la fois les premières données (D1) et des secondes données (D2) indicatives du retour respiratoire dans le sang dans des modèles physiologiques (MOD) descriptifs au moins d'une mécanique ventilatoire, et/ou d'un échange de gaz dans les poumons du patient, les modèles physiologiques comprenant un nombre de paramètres de modèle (MOD_P), et
**caractérisé en ce que**
le moyen de commande est en outre agencé pour simuler l'effet sur un ou plusieurs paramètres de modèle (MOD_P) choisis parmi des paramètres de mécanique ventilatoire, des paramètres d'échange de gaz, des paramètres sang-acide-base, des paramètres de pulsion respiratoire et des paramètres de circulation des modèles physiologiques pour une valeur suggérée du réglage de pression expiratoire positive (PEEP) pour le moyen de ventilation (11), et fournir ainsi une aide à la décision par rapport à ladite valeur PEEP suggérée.

18. Produit-programme d'ordinateur adapté pour permettre à un système d'ordinateur comprenant au moins un ordinateur ayant un moyen de stockage de données connecté à celui-ci de commander un système de ventilation mécanique selon le procédé de la revendication 17.
